# EUROPEAN PATENT APPLICATION

(11) **EP 4 775 562 A1**
(43) Date of publication of application: **15.07.2026**
(21) Application number: 24862977.6
(22) Date of filing: 25.06.2024
(51) Int. Cl.: C07C 29/80, C07C 29/04, C07C 31/10

(54) **METHOD OF PREPARING ISOPROPYL ALCOHOL**

(30) Priority: 08.09.2023 KR 20230119579
(71) Applicant: LG CHEM, LTD., Seoul 07336 (KR)
(72) Inventor: HWANG, Sung June, Daejeon 34122 (KR); LEE, Sung Kyu, Daejeon 34122 (KR); JANG, Kyung Soo, Daejeon 34122 (KR)
(74) Representative: Goddar, Heinz J.
(86) International application number: PCT/KR2024/008759
(87) International publication number: WO 2025/053394

(57) **Abstract**

The present invention provides a method of preparing isopropyl alcohol (IPA), and the method may include (S1) preparing a crude isopropyl alcohol (IPA) product obtained by performing gas purification and isopropyl alcohol purification on a reaction product of propylene and water; (S2) feeding the crude IPA product to a first section of a dividing wall distillation column including the first section, a second section, a top section, and a bottom section; and (S3) in the dividing wall distillation column, separating water and low boiling point organic materials contained in the crude IPA product to the top section, separating high boiling point organic and inorganic materials contained in the crude IPA product to the bottom section, and obtaining purified isopropyl alcohol in a liquid phase or a vapor phase in the second section.

## Description

### [Technical Field]

### CROSS-REFERENCE TO RELATED APPLICATION

The present application claims the benefit of priority to Korean Patent Application No. 10-2023-0119579, filed on September 8, 2023, the entire contents of which are incorporated herein by reference.

### Technical Field

The present invention relates to a method of preparing isopropyl alcohol, and more particularly, to a method of purifying high purity isopropyl alcohol by simultaneously removing organic and inorganic impurities from a crude isopropyl alcohol product.

### [Background Art]

Isopropyl alcohol (IPA) is used for various purposes including a solvent for a cleaning agent, a raw material for an industrial paint or reagent, a paint, an ink, or the like in the electronics industry such as the manufacture of a semiconductor, a liquid crystal display (LCD), or the like.

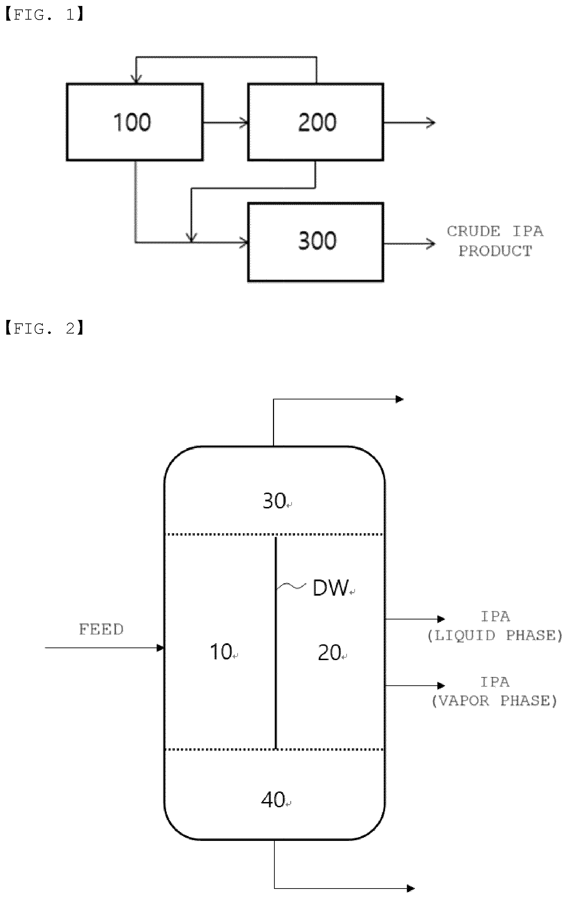

Such an isopropyl alcohol may be prepared by reacting propylene with water. Typically, as for isopropyl alcohol, referring to FIG. 1, a propylene monomer is reacted with water in a reaction unit 100 to obtain a reaction product containing an unreacted propylene monomer, unreacted water, and by-products such as n-propyl alcohol (NPA) and organic materials, along with IPA, the reaction product is transferred to a gas purification unit 200 to separate low boiling point gas components including the unreacted propylene monomer, and then, the reaction product from which the gas components are separated is fed to an IPA purification unit 300 including a plurality of distillation columns to remove the organic materials, NPA, and water, thereby obtaining a crude isopropyl alcohol product.

The crude isopropyl alcohol product obtained through the gas purification unit and the IPA purification unit may still contain trace amounts of the organic materials and water, and may contain, as impurities, inorganic materials (for example, metal components of Al, As, Fe, or Mg) present in reaction water or a reaction catalyst used as raw materials.

The inorganic impurities contained in the isopropyl alcohol may cause a decrease in semiconductor yield when used for semiconductor cleaning, and therefore, the inorganic impurities are required to be managed at a level of parts per billion (ppb) or parts per trillion (ppt) in the final product depending on the type of impurity.

Conventionally, methods of removing inorganic materials from a crude isopropyl alcohol product subjected to a distillation process using a filtration means equipped with a filter or a metal ion adsorption means have been performed.

However, when a content of the inorganic materials in the crude isopropyl alcohol product is excessive, it may be difficult to remove the inorganic materials to a level required in the semiconductor filed simply by applying the filtration means or the adsorption means.

### [Disclosure]

### [Technical Problem]

In order to solve the problems mentioned in the background art, an object of the present invention is to provide a method of purifying high purity isopropyl alcohol by effectively and simultaneously removing organic materials and inorganic materials remaining in a crude isopropyl alcohol product using a dividing wall distillation column.

### [Technical Solution]

In one general aspect, a method of preparing isopropyl alcohol includes:
(S1) preparing a crude isopropyl alcohol (IPA) product obtained by performing gas purification and isopropyl alcohol purification on a reaction product of propylene and water;
(S2) feeding the crude IPA product to a first section of a dividing wall distillation column including the first section, a second section, a top section, and a bottom section; and
(S3) in the dividing wall distillation column, separating water and low boiling point organic materials contained in the crude IPA product to the top section, separating high boiling point organic and inorganic materials contained in the crude IPA product to the bottom section, and obtaining purified isopropyl alcohol in a liquid phase or a vapor phase to the second section.

In the method, when the crude IPA product contains 50 ppb or less of the inorganic materials based on the total weight of the crude IPA product, the purified isopropyl alcohol may be obtained in a liquid phase in the second section of the dividing wall distillation column, and when the crude IPA product contains more than 50 ppb to 100 ppb of the inorganic materials based on the total weight of the crude IPA product, the purified isopropyl alcohol may be obtained in a vapor phase in the second section of the dividing wall distillation column.

In the second section of the dividing wall distillation column, a stage from which the purified isopropyl alcohol in the vapor phase is discharged is lower than a stage from which the purified isopropyl alcohol in the liquid phase is discharged.

### [Advantageous Effects]

According to the present invention, the IPA purification efficiency may be improved by simultaneously removing the organic and inorganic impurities contained in the crude IPA product through the application of the dividing wall distillation column.

In addition, in the present invention, the phase and discharge stage of the final IPA discharged from the dividing wall distillation column are controlled according to the content level of the inorganic materials contained in the crude IPA product, such that the inorganic material removal efficiency may be improved while realizing energy savings.

That is, when the content of the inorganic materials contained in the crude IPA product is 50 ppb or less, the final IPA is discharged in a liquid phase, such that it is possible to satisfy the inorganic material residual level required in the semiconductor field while minimizing energy consumption.

Meanwhile, when the content of the inorganic materials contained in the crude IPA product is more than 50 ppb, the final IPA is discharged in a vapor phase to separate the inorganic materials in the form of ions or precipitates, such that it is possible to overcome the limitations of inorganic material removal by the existing filtration means or adsorption means.

### [Description of Drawings]

FIG. 1 illustrates a typical process of preparing a crude isopropyl alcohol (IPA) product.
FIG. 2 schematically illustrates obtaining IPA purified with high purity from a crude IPA product in a liquid phase or a vapor phase by applying a dividing wall distillation column (dividing wall column (DWC)) in an embodiment of the present invention.

### [Best Mode]

The terms and words used in the description and claims of the present invention are not to be construed limitedly as having general or dictionary meanings but are to be construed as having meanings and concepts meeting the technical idea of the present invention, based on a principle that the inventors are able to appropriately define the concepts of terms in order to describe their own inventions in the best mode.

The meaning "including" or "containing" used in the present application concretely specifies a specific property, region, integer, step, operation, element, and/or component, and does not exclude addition of another specific property, region, integer, step, operation, element, and/or component.

The term "stream" used in the present application may refer to a flow of a fluid in a process, and may also refer to a fluid flowing through a pipe itself. Specifically, the stream may refer to both a fluid flowing through a pipe connecting respective devices to each other itself and a flow of a fluid. In addition, the fluid may include any one or more components of gas, liquid, and solid.

The term "top" used in the present application may refer to a point at a height of 0 to 20% from the top of a device to a lower portion, that is, the uppermost portion, unless otherwise specified. In addition, the term "bottom" may refer to a point at a height of 80 to 100% from the top of the device to the lower portion, that is, the lowermost portion.

In addition, the "pressure" referred to in the present application refers to gauge pressure measured based on atmospheric pressure.

An embodiment of the present invention relates to a method of preparing isopropyl alcohol (IPA). Hereinafter, the method of preparing IPA of the present invention will be described in detail with reference to the drawings.

First, a crude isopropyl alcohol (IPA) product is prepared (S1).

A commercially available crude IPA product may be purchased, or the crude IPA product may be prepared by a method typical in the art.

Referring to FIG. 1, a propylene monomer is reacted with water in a reaction unit 100 to obtain a reaction product containing an unreacted propylene monomer, unreacted water, and by-products such as n-propyl alcohol (NPA) and organic materials, along with IPA, and then the reaction product is transferred to a gas purification unit 200 including an absorption tower, a gas purification tower, and the like to separate low boiling point gas components including the unreacted propylene monomer. The reaction product (containing IPA, NPA, water, and organic materials) from which the gas components are separated is fed to an IPA purification unit 300, and the organic materials, NPA, and water are removed by a purification process performed in a plurality of distillation columns, thereby obtaining a crude isopropyl alcohol product.

Operating conditions of the gas purification unit and the IPA purification unit may be appropriately selected from the range commonly applied in the art, and there is no particular limitation.

The crude IPA product is obtained by feeding the reaction product of propylene and water to the gas purification unit and the IPA purification unit to separate gas components and organic materials, but the crude IPA product may still contain trace amounts of the organic materials and water. In addition, inorganic materials (for example, metal components of Al, As, Fe, or Mg) present in reaction water or a reaction catalyst used as raw materials may be contained as impurities.

For example, the crude IPA product may contain, based on the total weight of the crude IPA product, 0.08 wt% or less of water and low boiling point organic materials (for example, isopropyl ether, acetone, and the like), 0.05 wt% or less of high boiling point organic materials (for example, n-propyl alcohol, hexanol, and the like), and 100 ppb or less of inorganic materials (for example, metal components of Al, As, Fe, Mg, and the like), as impurities. The type and content of the impurities contained in the crude IPA product may vary depending on various environments involved in the reaction and purification processes.

In order to simultaneously remove trace amounts of water, organic materials, and inorganic materials contained in the crude IPA product, in the present invention, highly purified IPA is prepared using a dividing wall distillation column (dividing wall column (DWC)). The dividing wall distillation column that may be used in the present invention may have a structure commonly used in a petrochemical process without particular limitations.

As schematically illustrated in FIG. 2, the dividing wall distillation column used in the present invention may include a first section 10, a second section 20, a top section 30, and a bottom section 40 internally divided by a dividing wall (DW). The dividing wall DW may be installed at 30% to 70% or 35% to 65% of the total number of stages in the inner center of the distillation column, but is not limited thereto. As the middle section including the dividing wall DW, the first section 10 refers to a preliminary separation section, and the second section 20 refers to a main separation section. Meanwhile, the top section 30 and the bottom section 40 refer to the uppermost and lowermost sections of the distillation column that do not include the dividing wall DW.

In the first section 10, the second section 20, the top section 30, and the bottom section 40 divided by the dividing wall DW, a structure in which a porous plate or a grid-type tray and a packing bed obtained by filling packing on the porous plate or the grid-type tray in a layer having an appropriate height are formed may be installed in multiple stages. At each stage of the structure, heat and mass transfer occurs as a vapor phase stream that rises upward and a liquid phase stream that descends downward come into contact with each other, and as a result, a process in which some of heavy components condense and flow to the lower portion and uncondensed vapor continues to rise to the upper portion is performed continuously. The stage and size of the structure are not particularly limited, and may be set based on the theoretical stage inferred from a distillation curve considering a composition of a feed stream. In addition, the dividing wall distillation column may be provided with a reboiler that transfers heat to a stream discharged from the bottom section 40 and a condenser that converts a stream discharged from the top flow 30 to a liquid phase.

In the dividing wall distillation column having such a structure, the crude IPA product prepared in the previous step is fed as a feed to the first section 10, which is the preliminary separation section (S2).

The first section 10 may include one feeding port, and the feeding port may be located, for example, at a stage corresponding to 30% to 70% or 35% to 65% from an upper portion of the first section. In this case, a location of the feeding port may be selected in consideration of the type and content of impurities contained in the crude IPA product, the purity and residual amount of impurities of the desired final IPA, energy consumption, and the like.

In the first section 10, preliminary separation of the crude IPA product may be performed, and among the separated components, relatively low boiling point components may be introduced into the top section 30, and relatively high boiling point components may be introduced into the bottom section 40.

The second section 20 is the main separation section, where additional separation is performed according to the boiling points of the introduced components, and as in the first section 10, among the separated components, relatively low boiling point components may be introduced into the top section 30, relatively high boiling point components may be introduced into the bottom section 40, and middle boiling point components may be separated and discharged as a side stream of the second section 20.

As a result, while the crude IPA product fed to the dividing wall distillation column is subjected to the distillation process by continuous vapor-liquid contact in each stage included in the first section 10 for the preliminary separation and the second section 20 for the main separation, relatively low boiling point organic materials may rise in a vapor state and may be discharged from the top section 30, high boiling point organic and inorganic materials, which are relatively heavy components, may descend in a condensed state and may be discharged from the bottom section 40, and isopropyl alcohol from which the organic and inorganic materials are separated may be obtained in the side of the second section 20. Additionally, the discharge from the top section 30 may contain a trace amount of water contained in the crude IPA product.

The components discharged from the top section 30 may include water and low boiling point organic materials such as isopropyl ether and acetone, and may pass through the condenser, and then, some of the components may be discharged, and the remainder may be refluxed to the top section in a liquid phase.

For efficient separation of these low boiling point organic materials, the top section 30 may be operated at a temperature of 70 to 140°C or 75 to 110°C and a pressure of 0 to 5 kg/cm²·g or 0 to 2 kg/cm²·g.

The high boiling point organic materials discharged from the bottom section 40 may include n-propyl alcohol, hexanol, or a mixture thereof, and the inorganic materials separated to the bottom section of the dividing wall distillation column may include one or more metal components selected from Al, As, Fe, and Mg. The discharge from the bottom section 40 may pass through the reboiler, and then, a part of the discharge may be discharged, and the remainder may be refluxed to the bottom section in a vapor phase.

For efficient separation of these high boiling point organic and inorganic materials, the bottom section 30 may be operated at a temperature of 75 to 150°C or 80 to 120°C and a pressure of 0 to 6 kg/cm²·g or 0 to 3 kg/cm²·g.

As such, when a dividing wall distillation column is applied, organic and inorganic impurities contained in the crude IPA product may be simultaneously removed.

In an embodiment of the present invention, a discharge phase and a discharge location of the side stream, that is, purified IPA, in the second section 20 of the dividing wall distillation column may be selected depending on the composition of the crude IPA product fed to the first section 10.

When the content of the inorganic materials contained in the crude IPA product is trace, for example, 50 ppb or less, the final purified IPA may be obtained in a liquid phase through a discharge port for the side stream in the second section 20.

Specifically, the liquid phase IPA may be obtained by discharging the liquid collected in a collector tray installed at a stage located at 20% to 70% or 35% to 65% from an upper portion of the second section 20 of the dividing wall distillation column. When the discharge location is satisfied, the separation efficiency of the dividing wall distillation column may be maximized, such that energy consumption may be minimized and the residual levels of organic and inorganic materials required in the semiconductor field may be satisfied. Energy consumption may be reduced. In addition, the discharged liquid may satisfy the inorganic material residual level required in the semiconductor field since it is a purified product of a feed containing trace amounts of inorganic materials.

When the location where the liquid phase IPA is discharged is higher than 20% from the upper portion of the second section 20, the residual amount of the low boiling point organic materials in the final product may increase, and when the location where the liquid phase IPA is discharged is lower than 70% from the upper portion of the second section 20, the residual amount of the high boiling point organic materials in the final product may increase.

Meanwhile, when the content of the inorganic materials contained in the crude IPA product is more than 50 ppb, the final purified IPA may be obtained in a vapor phase through the discharge port for the side stream in the second section 20. Specifically, the vapor phase IPA may be obtained through a nozzle that provides a path through which vapor rises at a stage located at 40% to 80% or 45% to 75% from the upper portion of the second section of the dividing wall distillation column.

That is, the stage from which the vapor phase IPA is discharged should be lower than the stage from which the liquid phase IPA is discharged, and when this is satisfied, the inorganic materials contained in the stream may remain in the form of ions or precipitates in the liquid and may be separated from IPA discharged in a vapor phase, and the presence of the organic impurities in IPA discharged in a vapor phase may be minimized, satisfying the level required in the semiconductor field.

When the location where the vapor phase IPA is discharged is higher than 40% from the upper portion of the second section 20, the residual amount of the low boiling point organic materials in the vapor phase IPA may increase, and when the location where the vapor phase IPA is discharged is lower than 80% from the upper portion of the second section 20, the residual amounts of both the organic and inorganic materials in the vapor phase IPA may increase.

In an embodiment of the present invention, in the dividing wall distillation column, a flow rate of the purified IPA in the liquid phase or vapor phase discharged through the side stream of the second section 20 may be 95 wt% or more of the total flow rate of the crude IPA product fed to the first section 10.

In addition, in the purified IPA in the liquid phase or vapor phase, based on the weight of the purified IPA, a residual amount of the low boiling point organic materials is less than 50 ppm, a residual amount of the high boiling point organic materials is less than 20 ppm, and a residual amount of the inorganic materials is less than 50 ppb, satisfying the levels required in the semiconductor field.

According to the present invention as described above, the organic and inorganic impurities contained in the crude IPA product may be simultaneously removed by applying the dividing wall distillation column, and the phase and discharge stage of the final IPA discharged from the dividing wall distillation column are controlled according to the content level of the inorganic materials contained in the crude IPA product, such that the IPA purification efficiency may be improved while realizing energy savings.

That is, when the content of the inorganic materials contained in the crude IPA product is 50 ppb or less, the final IPA is discharged in a liquid phase, such that it is possible to satisfy the organic and inorganic material residual level required in the semiconductor field while minimizing energy consumption.

Meanwhile, when the content of the inorganic materials contained in the crude IPA product is more than 50 ppb, the final IPA is discharged in a vapor phase to separate the inorganic materials in the form of ions or precipitates, such that it is possible to overcome the limitations of inorganic material removal by the existing filtration means or adsorption means.

### [Mode for Invention]

Hereinafter, the present invention will be described in more detail with reference to Examples. However, the following Examples are provided for illustrating the present invention. It is apparent to those skilled in the art that various modifications and alterations may be made without departing from the scope and spirit of the present invention, and the scope of the present invention is not limited thereto.

In the following Examples and Comparative Examples, the method according to the present invention was simulated using the commercial process simulation program Aspen Plus. As the constants required for the simulation, the values stored in the program, the values described in the literature, and the like were used.

### Examples:

High purity IPA was purified from a crude IPA product using the dividing wall distillation column of FIG. 2.

First, a crude IPA product containing impurities was prepared with the composition as shown in Table 1, the crude IPA product was fed to a first section 10 of a dividing wall distillation column (DWC), a separation process was performed under the conditions shown in Table 1, and each stream was discharged from each of a top section 30, a bottom section 40, and a second section 20. The content of residual impurities contained in liquid phase or vapor phase IPA discharged from the second section 20 and the total amount of energy consumed in the dividing wall distillation column were measured and shown in Table 1.

**[Table 1]**

| | Crude IPA product composition | | | DWC separation conditions¹⁾ | | | Purified IPA composition | | | Amount of energy used²⁾ | Whether impurity residual level is passed |
|---|---|---|---|---|---|---|---|---|---|---|---|
| | Water/low boiling point organic naterial content (ppm) | High boiling point organic naterial content (ppm) | Inorganic material content (ppb) | Feed stage (%) | Stage from which liquid phase IPA is discharged (%) | Stage from which vapor phase IPA is discharged (%) | Water/low boiling point organic naterial content (ppm) | High boiling point organic naterial content (ppm) | Inorganic naterial content (ppb) | | |
| Example 1 | 450 | 250 | 35 | 50 | 40 | | 35 | 19 | 34 | 1.02 | ○ |
| Example 2 | 450 | 250 | 65 | 50 | | 60 | 43 | 18 | 28 | 1.28 | ○ |
| Example 3 | 450 | 250 | 35 | 50 | 80 | | 21 | 66 | 34 | 1.1 | K |
| Example 4 | 450 | 250 | 65 | 50 | | 20 | 78 | 14 | 22 | 1.35 | K |
| Example 5 | 450 | 250 | 65 | 50 | 40 | | 35 | 19 | 63 | 1 | K |
| 1) In the DWC, the location of the feed stage is based on a height from the upper portion (0%) of the first section to the lower portion (100%) of the first section, and the location of the stage from which the liquid phase and the vapor phase are discharged is based on a height from the upper portion (0%) of the second section to the lower portion (100%) of the second section. | | | | | | | | | | | |
| 2) The amount of energy used is expressed as a relative ratio based on the value of Example 5. | | | | | | | | | | | |

From Table 1, it is confirmed that, based on the content of the inorganic materials (50 ppb) contained in the crude IPA product, the phase and stage of the final IPA discharged from the dividing wall distillation column affect the content of residual impurities and the amount of energy used.

Specifically, in Example 1, the crude IPA product having a content of inorganic materials of less than 50 ppb was fed to the stage located at 50% of the first section in the dividing wall distillation column and the purified IPA was discharged in a liquid phase at the location corresponding to 40% of the second section, and as a result, the energy consumption was low, and the residual level of impurities required in the semiconductor field was satisfied.

In Example 3, the crude IPA product having a content of inorganic materials of less than 50 ppb was fed to the stage located at 50% of the first section and the purified IPA was discharged in a liquid phase at the location corresponding to 80% of the second section, and as a result, the residual amount of low boiling point organic materials decreased, but the residual amount of high boiling point organic materials increased, ultimately failing to satisfy the residual level of impurities.

In Example 2, the crude IPA product having a content of inorganic materials of more than 50 ppb was fed to the stage located at 50% of the first section and the purified IPA was discharged in a vapor phase at the location corresponding to 60% of the second section, and as a result, the energy consumption slightly increased, but the removal rate of the inorganic materials contained in the feed was excellent, satisfying the residual level of the impurities.

In Example 4, the crude IPA product having a content of inorganic materials of more than 50 ppb was fed to the stage located at 50% of the first section and the purified IPA was discharged in a vapor phase at the location corresponding to 20% of the second section, and as a result, the energy consumption increased, and the residual amount of the low boiling point organic materials was high, ultimately failing to satisfy the residual level of impurities.

Meanwhile, in Example 5, in the case of the crude IPA product having a content of inorganic materials of more than 50 ppb, the purified IPA was discharged in a liquid phase at the location corresponding to 40% of the second section, and as a result, although it was advantageous in terms of energy consumption compared to Example 2 in which vapor phase discharge was performed, the removal rate of the inorganic materials was poor, failing to satisfy the residual level of impurities.

### [Detailed Description of Main Elements]

100: Reaction unit
200: Purification unit
300: IPA purification unit
DW: Dividing wall
10: First section
20: Second section
30: Top section
40: Bottom section

## Claims

1. A method of preparing isopropyl alcohol, the method comprising:
(S1) preparing a crude isopropyl alcohol (IPA) product obtained by performing gas purification and isopropyl alcohol purification on a reaction product of propylene and water;
(S2) feeding the crude IPA product to a first section of a dividing wall distillation column including the first section, a second section, a top section, and a bottom section; and
(S3) in the dividing wall distillation column, separating water and low boiling point organic materials contained in the crude IPA product to the top section, separating high boiling point organic materials and inorganic materials contained in the crude IPA product to the bottom section, and obtaining purified isopropyl alcohol in a liquid phase or a vapor phase to the second section.

2. The method of claim 1, wherein the crude IPA product contains, based on the total weight of the crude IPA product, 0.08 wt% or less of the low boiling point organic materials, 0.05 wt% or less of the high boiling point organic materials, and 100 ppb or less of the inorganic materials, as impurities.

3. The method of claim 1, wherein when the crude IPA product contains 50 ppb or less of the inorganic materials based on the total weight of the crude IPA product, the purified isopropyl alcohol is obtained in a liquid phase in the second section of the dividing wall distillation column.

4. The method of claim 1, wherein when the crude IPA product contains more than 50 ppb to 100 ppb of the inorganic materials based on the total weight of the crude IPA product, the purified isopropyl alcohol is obtained in a vapor phase in the second section of the dividing wall distillation column.

5. The method of claim 1, wherein in the second section of the dividing wall distillation column, a stage from which the purified isopropyl alcohol in the vapor phase is discharged is lower than a stage from which the purified isopropyl alcohol in the liquid phase is discharged.

6. The method of claim 1, wherein the crude IPA product is fed to a stage located at 30% to 70% from an upper end portion of the first section of the dividing wall distillation column.

7. The method of claim 1, wherein the purified isopropyl alcohol in the liquid phase is discharged from a stage located at 20 to 70% from an upper end portion of the second section of the dividing wall distillation column.

8. The method of claim 1, wherein the purified isopropyl alcohol in the vapor phase is discharged from a stage located at 40 to 80% from an upper portion of the second section of the dividing wall distillation column.

9. The method of claim 1, wherein the low boiling point organic materials separated to the top section of the dividing wall distillation column include isopropyl ether, acetone, or a mixture thereof.

10. The method of claim 1, wherein the high boiling point organic materials separated to the bottom section of the dividing wall distillation column include n-propyl alcohol, hexanol, or a mixture thereof, and
the inorganic materials separated to the bottom section of the dividing wall distillation column include one or more metal components selected from Al, As, Fe, and Mg.

11. The method of claim 1, wherein in the purified isopropyl alcohol in the liquid phase or the vapor phase obtained in the second section of the dividing wall distillation column, based on the weight of the purified isopropyl alcohol, a residual amount of the low boiling point organic materials is less than 50 ppm, a residual amount of the high boiling point organic materials is less than 20 ppm, and a residual amount of the inorganic materials is less than 50 ppb.
